# EUROPÄISCHE PATENTSCHRIFT

(11) **EP 3 711 801 B1**
(45) Veröffentlichungstag und Bekanntmachung des Hinweises auf die Patenterteilung: **06.07.2022**
(21) Anmeldenummer: 20173447.2
(22) Anmeldetag: 27.07.2016
(51) Int. Cl.: A61M 15/00, A61M 16/06, B65D 83/14, B65D 83/20, B65D 83/22

(54) **FLÜSSIGKEITSSPENDER, INSBESONDERE INHALATOR**
LIQUID DISPENSER, IN PARTICULAR INHALER
DISTRIBUTEUR DE LIQUIDE, EN PARTICULIER INHALATEUR

(43) Veröffentlichungstag der Anmeldung: 23.09.2020
(62) Teilanmeldung aus: 16181529.5
(73) Patentinhaber: Aptar Radolfzell GmbH, 78315 Radolfzell (DE)
(72) Erfinder: Greiner-Perth, Jürgen, 78244 Gottmadingen (DE); Schwarz, Jutta, 78333 Stockach (DE)
(74) Vertreter: Witte, Weller und Partner Patentanwälte mbB Stuttgart

(56) Entgegenhaltungen:
- EP-A1- 0 510 469
- WO-A1-00/24652
- WO-A1-00/66459
- WO-A1-90/08098
- WO-A1-03/037749
- WO-A1-2009/023210
- WO-A1-2011/147714
- DE-A1-102009 044 280
- DE-U1-202006 010 781
- FR-A1- 2 996 537
- JP-A- 2001 048 254
- JP-A- 2006 306 414
- US-A- 3 534 890
- US-A1- 2004 238 573
- US-A1- 2005 072 810
- US-A1- 2007 235 474
- US-A1- 2008 041 375
- US-A1- 2013 256 338
- US-A1- 2015 108 171

## Beschreibung

Die Erfindung betrifft einen Flüssigkeitsspender nach dem Oberbegriff des Anspruchs 1.

Aus dem Stand der Technik sind vielfältige Flüssigkeitsspender bekannt. Die Erfindung betrifft insbesondere, jedoch nicht ausschließlich, Flüssigkeitsspender sowie Austragvorrichtungen hierfür, die als mobile Inhalatoren dienen können.

Ein gattungsgemäßer Flüssigkeitsspender, insbesondere in Form eines Inhalators genannter Art, dient dem Zweck, Flüssigkeit in insbesondere vernebelter Form abzugeben, um hierdurch einen pharmazeutischen Zweck zu erfüllen. Umfasst sind jedoch auch baulich ähnliche kosmetische Spender.

Die bekannten Gestaltungen von Inhalatoren gehen üblicherweise mit einer hohen Komplexität und einer Vielzahl von Bauteilen einher, so dass eine Verwendung als Einweg-Produkt problematisch ist.

Ein Flüssigkeitsspender gemäß dem Oberbegriff des Anspruchs 1 ist aus der WO 03/037749 A1 bekannt. Weitere Spender sind aus der FR 2996537 A1, US 3534890 A, US 2015/0108171 A1, DE 102009044280 A1, JP 2001048254 A, WO 00/66459 A1, JP 2006306414 A, DE 202006010781 U1, WO 90/08098 A1, WO 2009/023210 A1, US 2005/0072810 A1, EP 0510469 A1, US 2013/0256338 A1, US 2004/0238573 A1, WO 00/24652 A1, WO 2011/147714 A1, US 2008/0041375 A1 und US 2007/0235474 A1 bekannt.

### AUFGABE UND LÖSUNG

Aufgabe der Erfindung ist es, bauliche Vereinfachungen zur Verfügung zu stellen, die einen bezüglich der Handhabung optimalen Flüssigkeitsspender bei geringen Herstellungskosten ermöglichen. Die Erfindung umfasst mehrere diesbezügliche Aspekte.

Bezüglich eines derzeit nicht beanspruchten Aspekts wird eine Austragvorrichtung für einen Flüssigkeitsspender zum Austrag kosmetischer oder pharmazeutischer Flüssigkeiten vorgeschlagen, die folgende Gestaltung aufweist.

Die Austragvorrichtung verfügt über zwei Gehäusebauteile, die mittels einer Gewindeeinrichtung in einer überlagerten axialen und rotativen Bewegung gegeneinander beweglich sind. Diese Gewindeeinrichtung weist an einem ersten der beiden Gehäusebauteile mindestens eine helixabschnittsförmige Nut auf.

Die Gewindeeinrichtung weist an einem zweiten der beiden Gehäusebauteile eine Gewindenocke auf, die in die Nut eingreift. Weiterhin ist am ersten Gehäuseteil eine axial erstreckte Einführungsnut vorgesehen, die an einem Mündungspunkt in die helixabschnittsförmige Nut mündet.

Gemäß diesem Aspekt ist demnach vorgesehen, dass zusätzlich zur gleichsam einen Gewindegang darstellende helixabschnittsförmigen Nut zusätzlich eine axial erstreckte Einführungsnut vorgesehen ist, die in die den Gewindegang darstellende Nut mündet. Dies vereinfacht die Montage der betroffenen Gehäusebauteile erheblich, da zum Zwecke der Montage ein axiales Fügen der beiden Gehäusebauteile ausreicht. Die Gewindenocke kann daher an einer Stirnseite des mit der Nut versehenen Gehäusebauteils zunächst in die axial erstreckte Einführungsnut eingeschoben werden und gelangt so bis zur gewindetypischen helixabschnittsförmigen Nut.

Obwohl im Rahmen dieser Beschreibung meist auf eine helixabschnittsförmige Nut sowie eine Einführungsnut Bezug genommen wird, können diese auch mehrfach vorgesehen sein. So können insbesondere zwei oder drei oder auch mehr helixabschnittsförmige Nuten, gegebenenfalls mit jeweils einer Einführungsnut, vorgesehen sein, um eine besonders saubere Führung der Gehäusebauteile gegeneinander zu gewährleisten.

Dies kann zum einen genutzt werden, um die beiden Gehäusebauteile in einer Art fest miteinander zu verbinden, wie es üblicherweise mit Gewindeeinrichtungen geschieht. Dies bedeutet, dass die beiden Gehäusebauteile im Lieferzustand des Austragkopfes als Teil eines Flüssigkeitsspenders in einer festen Relativlage angeordnet sind, die bestimmungsgemäß im Betrieb keine Änderung mehr erfährt. Bei einer solchen Gestaltung weist die helixabschnittsförmige Nut eine Formgebung auf, die gemeinsam mit der Gewindenocke zu einer Selbsthemmung im Zuge des auf das axiale Fügen folgenden Festdrehens führt.

Bevorzugt ist jedoch eine Gestaltung, bei der die beiden Gehäusebauteile im Betrieb des Spenders bestimmungsgemäß durch Relativverlagerung zueinander einen Betriebsparameter des Spenders ändern, insbesondere das Öffnen und Schließen eines Auslassventils bewirken.

In einem solchen Fall ist somit vorgesehen, dass die beiden Gehäusebauteile, die vorzugsweise einer Basis und einem Austragkopf des Spenders zugeordnet sind, durch eine überlagerte axiale und rotative Relativbewegung zueinander geeignet sind, insbesondere den Austrag von Flüssigkeit mittels des Spenders auszulösen und zu beenden. Eine solche Relativbeweglichkeit zur Steuerung des Austrags hat sich als bei der Benutzung sehr intuitiv und gut dosierbar herausgestellt. Insbesondere kann der Benutzer ein Atemstück am Austragkopf durch Umschließen mit den Lippen oder dergleichen fixieren und durch Drehen des mit der Basis drehfest verbundenen Flüssigkeitsspeichers den Austrag beginnen und beenden.

Der Mündungspunkt, an dem die axial erstreckte Nut in die helixabschnittsförmige Nut mündet, ist vorzugsweise an einem der Enden der helixabschnittsförmigen Nut vorgesehen. Wenn die Gewindenocke bei der Einführung in die Einführnut diesen Mündungspunkt erreicht, ist eine Relativbeweglichkeit innerhalb der helixabschnittsförmigen Nut nur noch in eine Richtung gegeben.

Vorzugsweise ist die Gewindenocke auch im Lieferzustand des Spenders am Mündungspunkt, also beim Übergang von der axial erstreckten Nut in die helixabschnittsförmige Nut, angeordnet. Um hier die Relativlage zu sichern, kann innerhalb der Nut eine Widerstandsgeometrie vorgesehen sein, im Bereich derer die Bewegung der Gewindenocke eines höheren Kraftaufwandes bedarf. Für den Benutzer wird somit das Verlassen dieser Endlage der Gewindenocke in der helixabschnittsförmigen Nut haptisch erfassbar.

Bezüglich eines derzeit nicht beanspruchten zweiten Aspekts wird eine Austragvorrichtung vorgeschlagen, die folgende Gestaltung aufweist, wobei die Gestaltung vorzugsweise auch die Hauptmerkmale der vorbeschriebenen Gestaltung aufweist.

Die Austragvorrichtung gemäß diesem zweiten Aspekt verfügt über zwei Gehäusebauteile, die mittels einer Gewindeeinrichtung in einer überlagerten axialen und rotativen Bewegung gegeneinander beweglich sind. Diese Gewindeeinrichtung weist an einem ersten der beiden Gehäusebauteile mindestens eine helixabschnittsförmige Nut auf.

In der Nut ist ein mit der Gewindenocke zusammenwirkender Blockierabschnitt vorgesehen, wobei ein Überdrücken der Gewindenocke über den Blockierabschnitt in einer Fügerichtung möglich ist und in einer Trennrichtung verhindert wird.

Der in der Nut angeordnete Blockierabschnitt sorgt dafür, dass die Relativbeweglichkeit der Gehäusebauteile zueinander bei Erreichen des Blockierabschnitts durch die Gewindenocke nur in eine Richtung möglich ist. Somit ist eine Trennung der zwei Gehäusebauteile nach initialem Einführen der Gewindenocke in die Nut nicht mehr möglich. Von besonderem Vorteil ist dies im Zusammenhang mit der bereits erläuterten axial erstreckten Einführungsnut. Es gestattet, alleine durch eine axiale Bewegung der beiden Gehäusebauteile diese untrennbar miteinander zu fügen.

Der Blockierabschnitt und/oder die Gewindenocke ist/sind vorzugsweise mit einer Rampe versehen, die derart ausgerichtet sind, dass sie vor dem Fügen in Richtung des jeweils anderen Elements weist und beim Fügen der Gehäusebauteile diese Rampe die Vorbeiführung der Nocke am Blockierabschnitt erleichtert.

Weiterhin ist/sind der Blockierabschnitt und/oder die Gewindenocke vorzugsweise mit einer Anschlagsfläche versehen, die derart ausgerichtet sind, dass sie nach dem Fügen in Richtung des jeweils anderen Elements weist und nach dem Fügen eine Trennung der Gehäusebauteile verhindert.

Somit wird die Vorbeiführung der Gewindenocke am Blockierabschnitt dadurch ermöglicht, dass der Blockierabschnitt und/oder die Gewindenocke vorzugsweise eine bezogen auf die Fügerichtung asymmetrische Formgebung aufweisen. So kann beispielsweise durch Rampen beidseitig am Blockierabschnitt und an der Gewindenocke erreicht werden, dass in Fügerichtung nur eine geringe zusätzliche Kraft erforderlich ist, um die Gewindenocke über den Blockierabschnitt zu drücken, während in entgegengesetzter Richtung die Anschlagsflächen, die vorzugsweise orthogonal zur Fügerichtung/Trennrichtung ausgerichtet sind, in Anlage aneinander kommen und eine Trennung deutlich erschweren oder sogar eine zerstörungsfreie Trennung der beiden Gehäusebauteile verhindern.

Ein besonderer Vorteil ergibt sich, wenn der Blockierabschnitt am Ende der Einführungsnut vorgesehen ist und eine in Fügerichtung und somit auf den Mündungspunkt der axial erstreckten Einführungsnut hinweisende Anschlagsfläche aufweist. Diese ist dann in etwa fluchtend zu einer seitlichen Begrenzungsfläche der helixabschnittsförmigen Nut anzuordnen, so dass der Blockierabschnitt gleichsam im Betrieb mit seiner Anschlagsfläche einen Teil der seitlichen Wandung der helixabschnittsförmigen Nut bildet.

Erfindungsgemäß wird ein Flüssigkeitsspender nach Anspruch 1 vorgeschlagen. Die Unteransprüche definieren bevorzugte Ausführungsbeispiele der Erfindung.

Der erfindungsgemäße Flüssigkeitsspender ist ein Flüssigkeitsspender zum Austrag kosmetischer oder pharmazeutischer Flüssigkeiten, insbesondere in Form eines Inhalators. Der Flüssigkeitsspender verfügt über einen als Druckspeicher ausgebildeten Flüssigkeitsspeicher sowie eine Austragvorrichtung zum Austrag von Flüssigkeit aus dem Flüssigkeitsspeicher.

Die Austragvorrichtung umfasst eine Basis und einen Austragkopf, der zur Betätigung einer zwischen dem Flüssigkeitsspeicher des Flüssigkeitsspenders und der Austragvorrichtung vorgesehenen Ventileinrichtung (Auslassventil) gegenüber der Basis in einer überlagerten axialen und rotativen Bewegung verlagerbar ist, wobei die überlagerte Relativbeweglichkeit durch eine Gewindeeinrichtung bewirkt wird, die zwischen der Basis und dem Austragkopf angeordnet ist.

Die Basis weist ein erstes Bauteil mit einem hülsenförmigen Führungsabschnitt auf und der Austragkopf weist ein zweites Bauteil mit einem hülsenförmigen Führungsabschnitt auf. Zwischen einer Außenfläche des Führungsabschnitts der Basis und einer Innenfläche des Führungsabschnitts des Austragkopfes ist die Gewindeeinrichtung vorgesehen. Der Austragkopf verfügt über ein Zuströmrohr, welches in den Führungsabschnitt der Basis hineinragt und dort mittels einer an der Basis vorgesehenen Gleithülse zentriert ist.

Die hülsenförmigen Führungsabschnitte an der Basis zum einen und am Austragkopf zum anderen gewährleisten eine stabile Führung und bilden gemeinsam eine Gewindeeinrichtung, die eine überlagerte axiale und rotative Beweglichkeit zwischen dem Austragkopf und der Basis definiert. Um im Bereich dieser Führungsflächen ein Verkanten oder ähnliche Störungen zu vermeiden, sind die hülsenförmigen Führungsabschnitte vorzugsweise mit einem relativ großen Durchmesser versehen. Dies führt jedoch dazu, dass das Zuführrohr am Austragkopf vergleichsweise ungenau zentriert ist, was bei der Montage zu Schwierigkeiten führen kann.

Durch die zusätzliche Gleithülse an der Basis, innerhalb derer das Zuführrohr des Austragkopfes schraubenförmig beweglich ist, kann dennoch gewährleistet werden, dass dieses Zuführrohr stets zentriert gehalten wird. Es hat sich herausgestellt, dass die Störungen in der Produktion beim Aufsetzen des Austragkopfes auf einen Flüssigkeitsspeicher durch diese zusätzliche Zentrierung deutlich verringert werden.

Da das Zuführrohr innerhalb der Gleithülse sowohl axial als auch rotativ beweglich sein muss, ist es von Vorteil, wenn das Zuführrohr eine kreisrunde Außenform aufweist und die Gleithülse eine entsprechende kreisrunde Innenform aufweist. Die Gleithülse ist als separate konzentrische Hülse zum hülsenförmigen Führungsabschnitt der Basis ausgebildet und mit diesem fest, insbesondere einstückig, verbunden. Der entsprechende radial erstreckte Verbindungsabschnitt liegt vorzugsweise in Form mehrerer speichenartiger Verbindungsabschnitte vor, die den basisseitigen Führungsabschnitt mit der Gleithülse verbinden.

Die Gleithülse ist vorzugsweise derart an der Basis und dort am hülsenförmigen Führungsabschnitt vorgesehen, dass das Zuströmrohr selbst in jener Endlage, in der es maximal weit aus der Gleithülse herausgezogen ist, immer noch durch diese hindurchragt. Insbesondere in dieser Relativstellung erfolgt vorzugsweise die Montage, so dass es erheblich ist, dass das Zuströmrohr sich bis unter das untere Ende der Gleithülse erstreckt.

Bezüglich eines derzeit nicht beanspruchten vierten Aspekts wird eine Austragvorrichtung vorgeschlagen, die folgende Gestaltung aufweist, wobei die Gestaltung vorzugsweise auch die Merkmale einer der vorbeschriebenen Gestaltungen aufweist.

Die Austragvorrichtung gemäß diesem vierten Aspekt verfügt über einen Austragkopf mit einer Austragöffnung, durch die hindurch die Flüssigkeit in eine umgebende Atmosphäre abgegeben werden kann. Der Austragkopf weist ein äußeres Gehäusebauteil und mindestens zwei innere Gehäusebauteile auf, wobei die beiden inneren Gehäusebauteile gemeinsam flüssigkeitsführende Abschnitte des Austragkopfes begrenzen, mittels derer Flüssigkeit zur Austragöffnung geleitet wird.

Die beiden inneren Gehäusebauteile sind zur isolierten Abdichtung der flüssigkeitsführenden Abschnitte in einer ersten Montagerichtung aneinander gedrückt. Gemeinsam sind die beiden inneren Gehäusebauteile in einer von der ersten Montagerichtung abweichenden zweiten Montagerichtung in das Außengehäuse eingeschoben, so dass das Außengehäuse eine Trennung der beiden inneren Gehäusebauteile unterbindet.

Die beschriebene Bauform stellt eine vorteilhafte Möglichkeit dar, wenn am Austragkopf mehrere innere Gehäusebauteile vorgesehen sind, die gemeinsam flüssigkeitsführende Bereiche begrenzen. Eine solche Gestaltung kann insbesondere geboten sein, wenn zusätzliche Bauteile in den durch die beiden inneren Gehäusebauteile begrenzten Fluidbereich eingesetzt werden sollen, da eine einstückige Gestaltung der inneren Gehäusebauteile dann nicht möglich ist. Zu diesen zusätzlichen Teilen können beispielsweise Filter sowie Austrittsdüsen, insbesondere in Form von Düsenplatten, gehören. Bei einer Gestaltung gemäß diesem Aspekt sind die beiden inneren Bauteile getrennte Bauteile, die ineinander eingeschoben oder aufeinander aufgeschoben werden. Eine konstruktiv mitunter schwierige sichere Fügung dieser Bauteile kann dadurch entfallen, dass der Verbund der beiden inneren Gehäusebauteile in das genannte äußere Gehäusebauteil eingeschoben wird und somit das äußere Gehäusebauteil die inneren Gehäusebauteile gleichsam in Art einer Klammer gegen Trennung sichert.

Eines der inneren Gehäusebauteile weist vorzugsweise einen Auslasskanalabschnitt auf, der nach Einschub der inneren Gehäusebauteile in das äußere der Gehäusebauteile fluchtend zu einer Durchbrechung des Außengehäuses angeordnet ist.

Weiterhin sind das Außengehäuse und mindestens eines der inneren Gehäusebauteile vorzugsweise derart aufeinander abgestimmt, dass es zu einem Einrasten des Verbundes der beiden inneren Gehäusebauteile kommt, wenn diese in das äußere Gehäusebauteil eingeschoben werden.

Weiterhin kann es insbesondere von Vorteil sein, wenn auf der Innenseite des Außengehäuses oder an der Außenseite eines oder beider innerer Gehäusebauteile Einführschrägen vorgesehen sind, die das Einführen des Verbundes aus den beiden inneren Gehäusebauteilen erleichtern und dafür sorgen, dass die inneren Gehäusebauteile im eingeschobenen Zustand fluiddicht aneinander angepresst werden.

Der erfindungsgemäße Flüssigkeitsspender ist vorzugsweise zur Aufnahme von maximal 500 ml ausgelegt, vorzugsweise zur Aufnahme von maximal 250 ml ausgebildet.

Der Flüssigkeitsspeicher ist vorzugsweise mit einer der folgenden Flüssigkeiten befüllt, die insbesondere vorzugsweise in Form eines Sprühnebels zum Zwecke der Inhalation ausgetragen werden: eine salzhaltige wässrige Lösung oder eine wässrige Lösung in Form einer Ringerlösung oder einer gepufferten Lösung oder eine wässrige Lösung mit mindestens einem der Zusätze Kohlenhydrate, ätherische Öle, Menthol und Pflanzenextrakte oder eine wässrige Lösung, enthaltend Vitamine, Spurenelemente, Mangan oder Zink, oder eine wässrige Lösung mit mindestens einem der Zusätze aus der Gruppe umfassend Zimtöl, Teebaumöl, Salbeiöl, Thymianöl, Zitronenmelissenöl.

Der Flüssigkeitsspender ist vorzugsweise als Inhalator ausgebildet und verfügt über ein Atemstück, welches als Mundstück oder als Atemmaske ausgestaltet ist.

### KURZBESCHREIBUNG DER ZEICHNUNGEN

Weitere Vorteile und Aspekte der Erfindung ergeben sich aus den Ansprüchen und aus der nachfolgenden Beschreibung eines bevorzugten Ausführungsbeispiels der Erfindung, das nachfolgend anhand der Figuren erläutert ist.
Fig. 1 und 2 zeigen einen erfindungsgemäßen Flüssigkeitsspender in einer Gesamtdarstellung sowie einer geschnittenen Darstellung.
Fig. 3A und 3B verdeutlichen die Relativbeweglichkeit des Austragkopfes und der Basis des Austragkopfes der Fig. 1 und 2 zum Zwecke des Öffnens und Schließens des Auslassventils.
Fig. 4 und 5 zeigen die für diese Relativbeweglichkeit verantwortliche Gewindeeinrichtung.
Fig. 6 zeigt die Wirkweise der Gleithülse an der Basis des erfindungsgemäßen Spenders.
Fig. 7A bis 7C zeigen die Montage des Austragkopfes des Spenders.

### DETAILLIERTE BESCHREIBUNG DES AUSFÜHRUNGSBEISPIELS

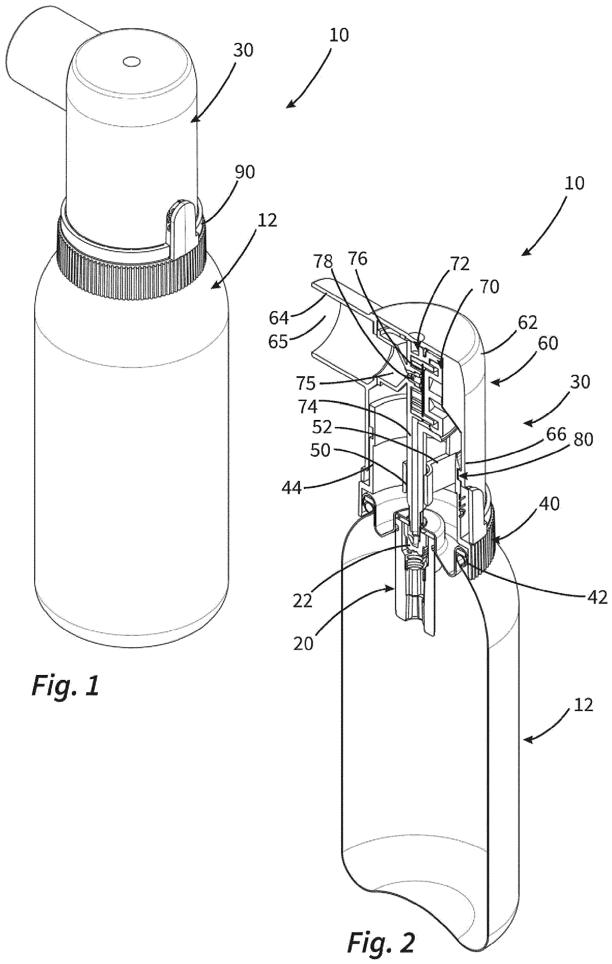

Die Fig. 1 und 2 zeigen einen erfindungsgemäßen Flüssigkeitsspender 10, der vorliegend als Inhalator ausgebildet ist, in einer Gesamtdarstellung sowie in einer geschnittenen Darstellung.

Der Flüssigkeitsspender 10 verfügt über eine Austragvorrichtung 30, die auf einen als Druckspeicher ausgebildeten Flüssigkeitsspeicher 12 aufgesetzt ist. Zwischen dem Flüssigkeitsspeicher 12 und der Austragvorrichtung 30 ist eine Ventileinrichtung 20 vorgesehen, die über einen durch axiales Niederdrücken verlagerbaren Ventilkörper 22 geöffnet werden kann und nach Wegfall der axialen Beaufschlagung durch eine Ventilfeder zurück in die geschlossene Ausgangslage gedrückt wird.

Die Austragvorrichtung 30 verfügt über eine Basis 40, die mittels einer Klemmgeometrie 42 drehfest am Flüssigkeitsspeicher 12 befestigt ist. Diese Basis 40 stellt einen hülsenförmigen Führungsabschnitt 44 zur Verfügung, an der ein Austragkopf 60 der Austragvorrichtung 30 beweglich geführt ist.

Der Austragkopf 60 weist ein Außengehäuse-Bauteil 62 auf, innerhalb dessen zwei Innenbauteile 70, 72 angeordnet sind, die ortsfest zum Außengehäuse-Bauteil 62 befestigt sind und gemeinsam einen Flüssigkeitspfad von der Ventileinrichtung 20 durch ein Zuströmrohr 74 bis zu einem Flüssigkeitsauslass 75 begrenzen. In dem durch die beiden Innenbauteile 70, 72 gemeinsam definierten Innenraum sind eine Düseneinheit 78 mit einer nicht näher dargestellten Düsenplatte sowie eine Filtereinheit 76 mit Membran angeordnet. Das Zuströmrohr 74 ragt bis zum Ventilkörper 22 und ist in der Lage, diesen zum Öffnen derVentileinrichtung kraftzubeaufschlagen.

Nach Entfernen eines Originalitätsabschnittes 90 ist der Austragkopf 60 gegenüber der Basis 40 beweglich. Der Austragkopf 60 kann dann in einer überlagerten rotativen und axialen Bewegung geführt gegenüber der Basis 40 verlagert werden, um hierdurch mittels des Zuströmrohrs 74 die Ventileinrichtung 20 zu öffnen und zu schließen.

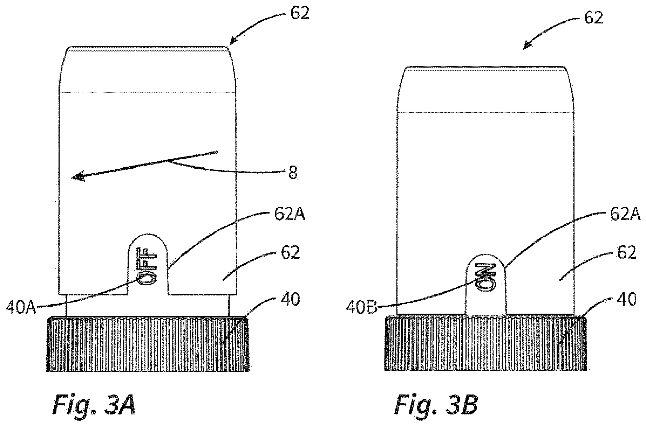

In Fig. 3A ist der Zustand des Flüssigkeitsspenders 10 mit geschlossener Ventileinrichtung 20 dargestellt. In diesem Zustand ist der Austragkopf 60 maximal gegenüber der Basis 40 beabstandet, so dass das Zuströmrohr 74 in seiner oberen Endlage angeordnet ist und daher den Ventilkörper 22 nicht aus seiner Schließlage nach unten drückt. Dieser geschlossene Zustand wird in einem Sichtfenster 62A mittels der Beschriftung "OFF" gekennzeichnet.

Ausgehend hiervon kann der Austragkopf 60 in Richtung des Pfeils 8 gegenüber der Basis 40 gleichzeitig verdreht und axial nach unten verlagert werden. Diese axiale Verlagerung bewirkt, dass das in Fig. 2 dargestellte Zuströmrohr 74 als Teil des Austragkopfes 60 nach unten verlagert wird und dadurch die Ventileinrichtung 20 öffnet. In der Praxis erfolgt diese Relativbewegung jedoch durch Bewegung des Flüssigkeitsspeichers mitsamt der Basis 40 gegenüber dem Austragkopf 60, dessen Atemstück 64 ein Benutzer mit den Lippen umschließt oder dessen Atemstück 64 mittels einer daran angesetzten Atemmaske, die an das Gesicht angedrückt wird, fixiert ist.

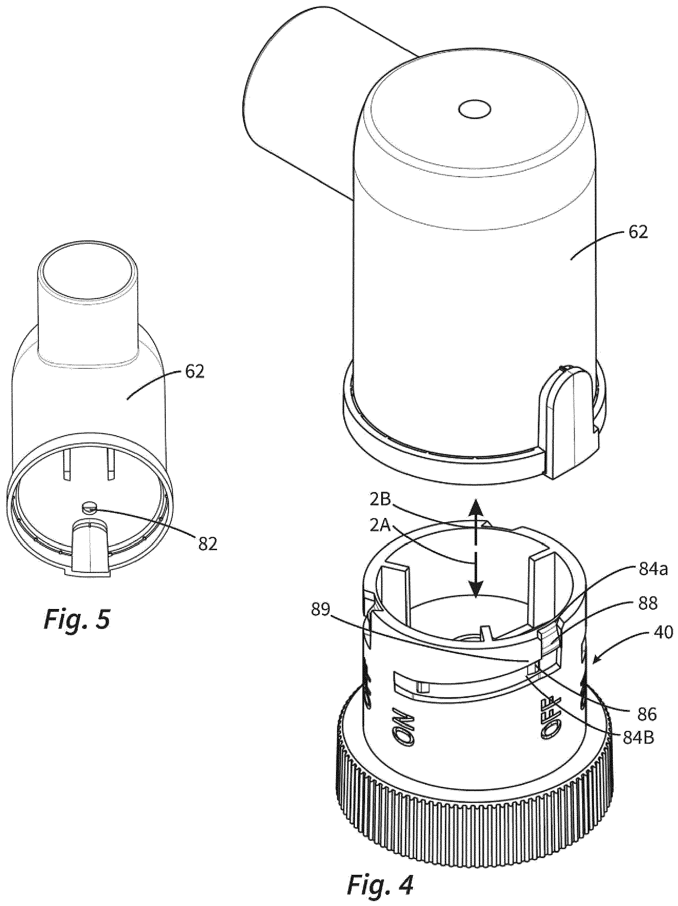

Die bauliche Gestaltung zur Erzielung der überlagerten Beweglichkeit sowie Besonderheiten in diesem Kontext werden anhand der Fig. 4 und 5 verdeutlicht.

Aus Fig. 4 ist ersichtlich, dass das Basisteil über eine helixabschnittsförmige Gewindenut 84B verfügt. Korrespondierend hierzu ist an der Innenseite des Außengehäuse-Bauteils 62 eine Gewindenocke 82 vorgesehen, die im Betrieb entlang der Gewindenut 84B verlagerbar ist, um in den Endlagen einen der Zustände der Fig. 3A und 3B einzunehmen.

Zum Zwecke einer einfachen Montage ist an der Basis 40 zusätzlich eine axial erstreckte Nut 84A vorgesehen, die bezogen auf Fig. 4 am rechten Ende in die helixabschnittsförmige Nut 84B mündet. Innerhalb dieser axial erstreckten Nut 84A ist ein Blockierabschnitt 88 vorgesehen, der nach oben weisend eine Einführschräge aufweist und nach unten weisend über eine Anschlagsfläche verfügt, die mit der oberen Begrenzungsfläche 89 der helixabschnittsförmigen Nut 84B fluchtet. Diese Nut 84A ermöglicht es, ausgehend von der Relativlage des Außengehäuse-Bauteils 62 zur Basis 40 der Fig. 4 im Zuge der Montage lediglich das Außengehäuse-Bauteil 62 in Montagerichtung 2A auf die Basis 40 aufzuschnappen, wobei hierdurch die Gewindenocke 82 bis in die helixabschnittsförmige Nut 84B gelangt. Sobald die Gewindenocke 82 über den Blockierabschnitt 88 hinübergedrückt ist, sind das Außengehäuse-Bauteil 62 und die Basis 40 sicher miteinander verbunden.

Die Gewindeeinrichtung 80 kann somit ohne rotative Bewegung im Zuge der Montage gefügt werden. Da es vorliegend gewünscht ist, dass die Gewindenocke 82 vor Inbetriebnahme nicht entlang der helixabschnittsförmigen Nut 84B versehentlich verlagert wird und zusätzlich der deaktivierte Zustand des Spenders später durch den Nutzer haptisch erfassbar sicherbar sein soll, ist zusätzlich eine Widerstandsgeometrie 86 in der Nut 84B vorgesehen, die ein Passieren der Gewindenocke 82 nur unter erhöhtem Kraftaufwand gestattet.

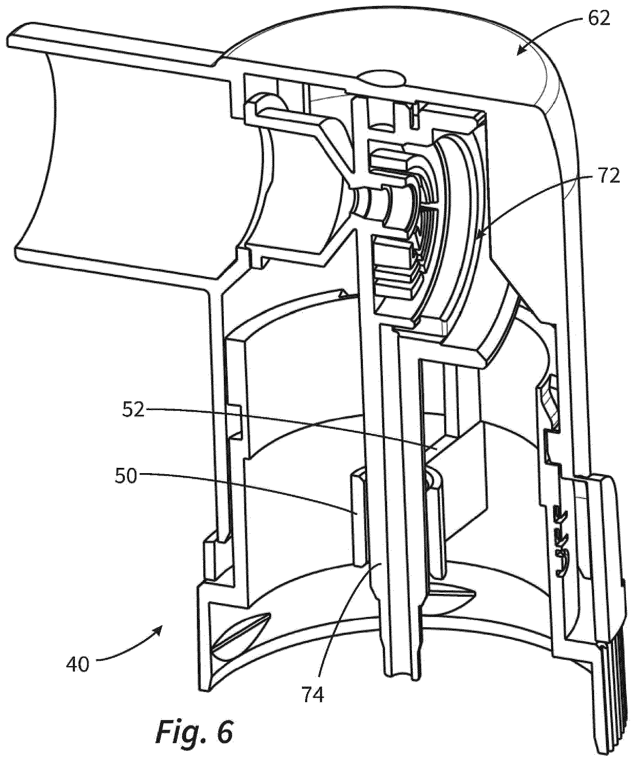

Fig. 6 verdeutlicht, wie der Austragkopf 60 an der Basis 40 geführt ist. Diese Führung erfolgt zum einen an den hülsenförmigen Abschnitten 44, 66 der Basis 40 und des Außengehäuse-Bauteils 62. Zusätzlich ist jedoch eine Gleithülse 50 vorgesehen, die über drei um jeweils 120° beabstandete speichenartige Verbindungsabschnitte 52 zentrisch im hülsenförmigen Abschnitt 44 der Basis 40 gehalten wird. Durch diese Gleithülse 50 hindurch ragt das Zuführrohr 74. Die Austragvorrichtung der Fig. 6 kann somit bei hoher Verfahrenssicherheit auf den Flüssigkeitsspeicher 12 und die Ventileinrichtung 20 aufgesetzt werden. Wäre die Gleithülse 50 nicht vorhanden, so wäre hier ein Blockieren aufgrund mangelnder Zentrierung zu befürchten.

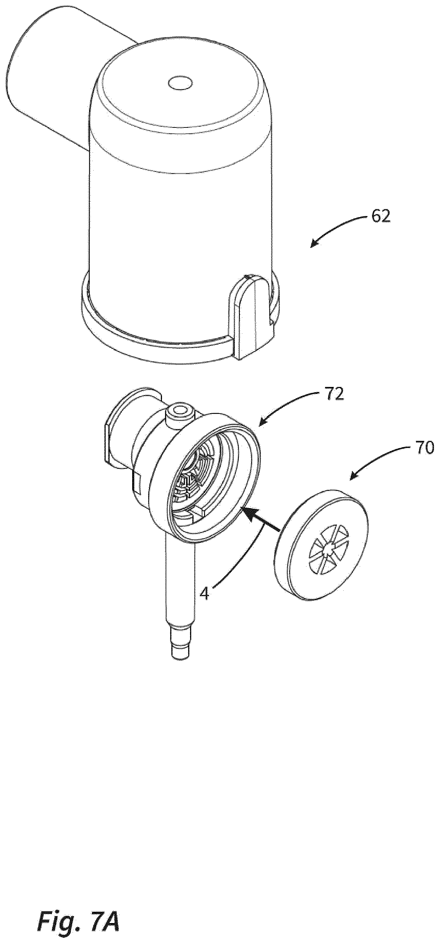

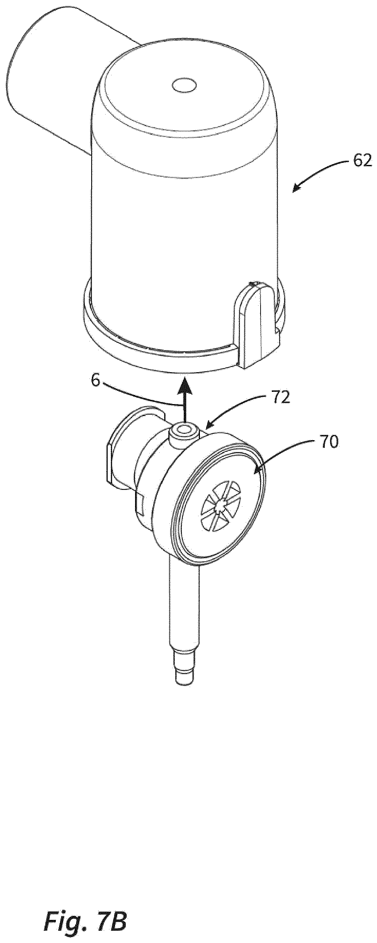

Die Montage der genannten Innenbauteile 70, 72 im Außengehäuse-Bauteil 62 wird anhand der Fig. 7A bis 7C verdeutlicht. Ausgehend von den drei genannten Einzelteilen wird zunächst das Innenbauteil 70, welches eine Art Stopfen darstellt, in einer ersten Montagerichtung 4 in das zweite Innenbauteil 72 eingeschoben, in welchem zuvor in nicht dargestellter Weise die Düseneinheit 78 und die Filtereinheit 76 eingesetzt wurden.

Anschließend wird der Verbund aus den beiden inneren Gehäusebauteilen 70, 72 gemeinsam in einer zweiten Montagerichtung 6 in das Außengehäuse-Bauteil 62 eingeschoben.

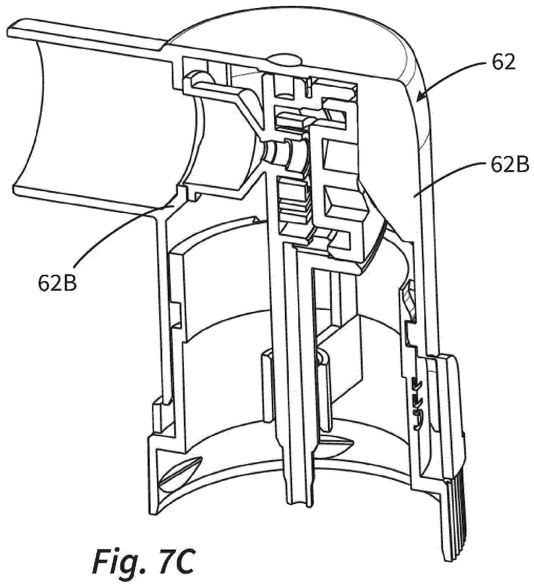

Wie Fig. 7C zeigt, sind die beiden Innenbauteile 70, 72 hierdurch im montierten Zustand im Außengehäuse-Bauteil 62 gleichsam eingeklemmt und hierdurch sicher gegen Trennen gesichert.

## Patentansprüche

1. Flüssigkeitsspender (10) zum Austrag kosmetischer oder pharmazeutischer Flüssigkeiten, insbesondere in Form eines Inhalators, mit den folgenden Merkmalen:
a. der Flüssigkeitsspender (10) verfügt über einen als Druckspeicher ausgebildeten Flüssigkeitsspeicher (12), und
b. der Flüssigkeitsspender (10) verfügt über eine Austragvorrichtung (30) zum Austrag von Flüssigkeit aus dem Flüssigkeitsspeicher (12), und
c. die Austragvorrichtung (30) umfasst eine Basis (40) und
d. die Austragvorrichtung umfasst einen Austragkopf (60), der zur Betätigung einer zwischen dem Flüssigkeitsspeicher (12) und der Austragvorrichtung (30) vorgesehenen Ventileinrichtung (20) gegenüber der Basis (40) in einer überlagerten axialen und rotativen Bewegung verlagerbar ist, und
e. die überlagerte Relativbeweglichkeit wird durch eine Gewindeeinrichtung (80) bewirkt, die zwischen der Basis (40) und dem Austragkopf (60) angeordnet ist, und
f. die Basis (40) weist ein erstes Bauteil mit einem hülsenförmigen Führungsabschnitt (44) auf,
g. der Austragkopf (60) weist ein zweites Bauteil (62) mit einem hülsenförmigen Führungsabschnitt (66) auf,
h. die Gewindeeinrichtung (80) ist zwischen einer Außenfläche des Führungsabschnitts (44) der Basis (40) und einer Innenfläche des Führungsabschnitts (66) des Austragkopfes (60) vorgesehen, und
i. der Austragkopf (60) verfügt über ein Zuströmrohr (74),
**dadurch gekennzeichnet, dass**
das Zuströmrohr (74) in den Führungsabschnitt (44) der Basis (40) hineinragt und dort mittels einer an der Basis (40) vorgesehenen Gleithülse (50) zentriert ist, wobei die Gleithülse (50) als separate konzentrische Hülse zum hülsenförmigen Führungsabschnitt der Basis ausgebildet und mit diesem Fest, insbesondere einstückig, verbunden ist.

2. Flüssigkeitsspender (10) nach Anspruch 1 mit dem Merkmal:
a. die Gleithülse (50) ist als vom hülsenförmigen Führungsabschnitt (44) der Basis getrennte Gleithülse (50) ausgebildet und mittels mindestens eines Verbindungsabschnitts (52), insbesondere mittels mehrerer speichenartiger Verbindungsabschnitte (52), mit dem Führungsabschnitt (44) der Basis (40) verbunden.

3. Flüssigkeitsspender (10) nach einem der Ansprüche 1 oder 2 mit den Merkmalen:
a. der hülsenförmige Führungsabschnitt (44) der Basis (40) und der hülsenförmige Führungsabschnitt (66) des Austragkopfes (60) sind gegeneinander zwischen zwei Endlagen axial relativbeweglich, und
b. die Gleithülse (50) und das Zuströmrohr (74) sind derart aufeinander abgestimmt, dass das Zuströmrohr in beiden Endlagen durch die Gleithülse (50) hindurchragt.

4. Flüssigkeitsspender (10) nach einem der vorherigen Ansprüche mit dem folgenden Merkmal:
a. der Flüssigkeitsspeicher (12) ist zur Aufnahme von maximal 500 ml ausgelegt, vorzugsweise von maximal 250 ml.

5. Flüssigkeitsspender (10) nach einem der vorherigen Ansprüche mit dem folgenden Merkmal:
a. der Flüssigkeitsspeicher (12) ist befüllt mit:
- einer salzhaltigen wässrigen Lösung oder
- einer wässrigen Lösung in Form einer Ringerlösung oder einer gepufferten Lösung oder
- einer wässrigen Lösung mit mindestens einem der Zusätze Kohlenhydrate, ätherische Öle, Menthol und Pflanzenextrakte oder
- einer wässrigen Lösung, enthaltend Vitamine, Spurenelemente, Mangan oder Zink, oder
- einer wässrigen Lösung mit mindestens einem der Zusätze aus der Gruppe umfassend Zimtöl, Teebaumöl, Salbeiöl, Thymianöl, Zitronenmelissenöl.

6. Flüssigkeitsspender (10) nach einem der vorherigen Ansprüche mit dem folgenden Merkmal:
a. der Flüssigkeitsspender (10) ist als Inhalator ausgebildet und verfügt über ein Atemstück (64), welches als Mundstück oder als Atemmaske ausgestaltet ist.

7. Flüssigkeitsspender (10) nach Anspruch 6 mit dem folgenden Merkmal:
a. das Atemstück (64) weist einen Kanalabschnitt auf, der in einer von einer Betätigungsrichtung des Austragskopfs abweichenden Richtung ausgerichtet ist und vorzugsweise mit dieser einen Winkel zwischen 45° und 135° einschließt, insbesondere vorzugsweise einen Winkel zwischen 70° und 110°.

## Claims

1. Liquid dispenser (10) for discharging cosmetic or pharmaceutical liquids, in particular in the form of an inhaler, having the following features:
a. the liquid dispenser (10) has a liquid reservoir (12) configured as a pressure reservoir, and
b. the liquid dispenser (10) has a discharge device (30) for discharging liquid from the liquid reservoir (12), and
c. the discharge device (30) comprises a base (40), and
d. the discharge device comprises a discharge head (60) which, for actuation of a valve mechanism (20) provided between the liquid reservoir (12) and the discharge device (30), is movable with respect to the base (40) in a superposed axial and rotational movement, and
e. the superposed relative movability is brought about by a thread mechanism (80) which is arranged between the base (40) and the discharge head (60), and
f. the base (40) has a first component with a sleeve-shaped guide portion (44),
g. the discharge head (60) has a second component (62) with a sleeve-shaped guide portion (66),
h. the thread mechanism (80) is provided between an outer surface of the guide portion (44) of the base (40) and an inner surface of the guide portion (66) of the discharge head (60), and
i. the discharge head (60) has an inflow tube (74),
**characterized in that**
the inflow tube (74) projects into the guide portion (44) of the base (40) and is centred there by means of a sliding sleeve (50) which is provided on the base (40), wherein the sliding sleeve (50) is configured as a separate concentric sleeve to the sleeve-shaped guide portion of the base and is connected fixedly, in particular integrally, thereto.

2. Liquid dispenser (10) according to Claim 1, having the following feature:
a. the sliding sleeve (50) is configured as a sliding sleeve (50) which is separated from the sleeve-shaped guide portion (44) of the base and is connected to the guide portion (44) of the base (40) by means of at least one connecting portion (52), in particular by means of a plurality of spoke-like connecting portions (52).

3. Liquid dispenser (10) according to either of Claims 1 and 2, having the following features:
a. the sleeve-shaped guide portion (44) of the base (40) and the sleeve-shaped guide portion (66) of the discharge head (60) are relatively movable axially with respect to each other between two end positions, and
b. the sliding sleeve (50) and the inflow tube (74) are coordinated with each other in such a manner that the inflow tube projects through the sliding sleeve (50) in both end positions.

4. Liquid dispenser (10) according to one of the preceding claims, having the following feature:
a. the liquid reservoir (12) is configured to receive a maximum of 500 ml, preferably a maximum of 250 ml.

5. Liquid dispenser (10) according to one of the preceding claims, having the following feature:
a. the liquid reservoir (12) is filled with:
- a saline aqueous solution or
- an aqueous solution in the form of a Ringer's solution or a buffered solution or
- an aqueous solution with at least one of the additives carbohydrates, essential oils, menthol and plant extracts or
- an aqueous solution containing vitamins, trace elements, manganese or zinc, or
- an aqueous solution with at least one of the additives from the group comprising cinnamon oil, tea tree oil, sage oil, thyme oil, or lemon balm oil.

6. Liquid dispenser (10) according to one of the preceding claims, having the following feature:
a. the liquid dispenser (10) is configured as an inhaler and has a respiration piece (64), which is configured as a mouthpiece or as a breathing mask.

7. Liquid dispenser (10) according to Claim 6, having the following feature:
a. the respiration piece (64) has a channel portion which is oriented in a direction deviating from an actuation direction of the discharge head and which preferably encloses with the latter an angle of between 45° and 135°, in particular preferably an angle of between 70° and 110°.

## Revendications

1. Distributeur de liquide (10) destiné à la délivrance de liquides cosmétiques ou pharmaceutiques, notamment sous la forme d'un inhalateur, ledit distributeur de liquide présentant les caractéristiques suivantes :
a. le distributeur de liquide (10) comporte un réservoir de liquide (12) réalisé sous la forme d'un réservoir sous pression, et
b. le distributeur de liquide (10) comporte un dispositif de délivrance (30) destiné à délivrer du liquide du réservoir de liquide (12), et
c. le dispositif de délivrance (30) comprend une base (40) et
d. le dispositif de délivrance comprend une tête de délivrance (60) qui est déplaçable par rapport à la base (40) suivant un mouvement axial et rotatif superposé afin d'actionner un mécanisme à clapet (20) prévu entre le réservoir de liquide (12) et le dispositif de délivrance (30), et
e. la mobilité relative superposée est réalisée par un mécanisme à pas de vis (80) qui est disposé entre la base (40) et la tête de délivrance (60), et
f. la base (40) comporte un premier composant pourvu d'une portion de guidage (44) en forme de manchon,
g. la tête de délivrance (60) comporte un deuxième composant (62) pourvu d'une portion de guidage (66) en forme de manchon,
h. le mécanisme à pas de vis (80) est prévu entre une surface extérieure de la portion de guidage (44) de la base (40) et une surface intérieure de la portion de guidage (66) de la tête de délivrance (60), et
i. la tête de délivrance (60) comporte un tube d'arrivée (74),
**caractérisé en ce que**
le tube d'arrivée (74) fait saillie dans la portion de guidage (44) de la base (40) où il est centré au moyen d'un manchon coulissant (50) prévu au niveau de la base (40), le manchon coulissant (50) étant conçu comme un manchon concentrique séparé par rapport à la portion de guidage de la base et étant relié à celle-ci de manière fixe, en particulier d'une seule pièce.

2. Distributeur de liquide (10) selon la revendication 1 présentant la caractéristique suivante :
a. le manchon coulissant (50) est conçu comme un manchon coulissant (50) séparé de la portion de guidage (44) en forme de manchon de la base et est relié à la portion de guidage (44) de la base (40) au moyen d'au moins une portion de liaison (52), notamment au moyen de plusieurs portions de liaison (52) en forme de rayons.

3. Distributeur de liquide (10) selon l'une des revendications 1 ou 2 présentant les caractéristiques suivantes :
a. la portion de guidage (44), en forme de manchon, de la base (40) et la portion de guidage (66), en forme de manchon, de la tête de délivrance (60) sont mobiles axialement l'une par rapport à l'autre entre deux positions d'extrémité, et
b. le manchon coulissant (50) et le tube d'arrivée (74) sont adaptés l'un à l'autre de manière à ce que le tube d'arrivée fasse saillie du manchon coulissant (50) dans les deux positions d'extrémité.

4. Distributeur de liquide (10) selon l'une des revendications précédentes présentant la caractéristique suivante :
a. le réservoir de liquide (12) est conçu pour recevoir un maximum de 500 ml, de préférence un maximum de 250 ml.

5. Distributeur de liquide (10) selon l'une des revendications précédentes présentant la caractéristique suivante :
a. le réservoir de liquide (12) est rempli :
- d'une solution aqueuse saline ou
- d'une solution aqueuse se présentant sous la forme d'une solution de Ringer ou d'une solution tamponnée ou
- d'une solution aqueuse comprenant au moins un des additifs glucides, huiles essentielles, menthol et extraits de plantes ou
- d'une solution aqueuse contenant des vitamines, des oligo-éléments, du manganèse ou du zinc, ou
- d'une solution aqueuse comprenant au moins un des additifs du groupe comprenant l'huile de cannelle, l'huile d'arbre à thé, l'huile de sauge, l'huile de thym, l'huile de mélisse.

6. Distributeur de liquide (10) selon l'une des revendications précédentes présentant la caractéristique suivante :
a. le distributeur de liquide (10) est conçu sous la forme d'un inhalateur et comporte un embout respiratoire (64) qui est réalisé sous la forme d'un embout buccal ou sous la forme d'un masque respiratoire.

7. Distributeur de liquide (10) selon la revendication 6 présentant la caractéristique suivante :
a. l'embout respiratoire (64) comporte une portion de canal qui est orientée dans une direction s'écartant d'une direction d'actionnement de la tête de délivrance et forme de préférence avec celle-ci un angle compris entre 45° et 135°, en particulier de préférence un angle compris entre 70° et 110°.
